# EUROPÄISCHE PATENTSCHRIFT

(11) **EP 0 439 871 B1**
(45) Veröffentlichungstag und Bekanntmachung des Hinweises auf die Patenterteilung: **31.05.1995**
(21) Anmeldenummer: 90250340.8
(22) Anmeldetag: 27.12.1990
(51) Int. Cl.: A61K 51/04

(54) **Myokardgängige Technetium-Komplexverbindungen und Verfahren zu deren Herstellung**
Myocardial-path technetium complex compounds and process for their production
Composés complexes de technetium du trajet myocardique et leur procédé de production

(30) Priorität: 29.12.1989 DD 336698
(43) Veröffentlichungstag der Anmeldung: 07.08.1991
(73) Patentinhaber: Forschungszentrum Rossendorf e.V., D-01314 Dresden (DE)
(72) Erfinder: Pietzsch, Hans-Jürgen, O-8312 Heidenau (DE); Spies, Hartmut, Dr., O-8019 Dresden (DE); Syhre, Rosemarie, Dr., O-8060 Dresden (DE); Seifert, Sepp, Dr., O-8019 Dresden (DE); Münze, Rudolf, Prof. Dr., O-8051 Dresden (DE); Beger, Jörg, Prof. Dr., O-9200 Freiberg (DE)
(74) Vertreter: Schramm, Matthias

(56) Entgegenhaltungen:
- EP-A- 0 163 294
- EP-A- 0 311 352
- EP-A- 0 337 654
- Nucl. Med. Biol. 16(3), 191 - 232, 1989
- Deutsch et al., Privatmitteilung
- J. Nucl. Med. 35(2), 334 - 341, 1994
- INORGANICA CHIMICA ACTA, Bd. 165, 22. Dezember 1989, Seiten 163-166, Elsevier Sequoia, Lausanne, CH; H.-J. PIETZSCH et al.: "Lipophilic technetium complexes VI. Neutral Oxotechnetium (V) complexes with monothiole/tridentate dithiole coordination"

## Beschreibung

Die Erfindung betrifft einfach kationische Komplexe des Technetiums die als potentielle Radiopharmaka bevorzugt im Herzmuskelgewebe angereichert werden.

Technetiumverbindungen werden als radioaktive Diagnostika zu Funktions- und Lokalisationsuntersuchungen des Myokards verwendet. Voraussetzung für erfolgreiche Pharmakonentwicklungen ist die Verfügbarkeit von Technetiumverbindungen, die aufgrund ihrer chemischen Eigenschaften die für den diagnostischen Zweck erforderlichen Bioverteilungsmuster aufweisen.

Herzmuskelaufnahme wurde bisher vorwiegend bei solchen Technetiumkomplexen gefunden, die positiv geladen sind und in denen der Komplexbildner organische Reste enthält. Kationische Technetiumkomplexe und deren Synthese unter Zusatz von Kupfersalzen werden z.B. in der Patentschrift EP 0 163 294 beschrieben. Darin finden sich jedoch keine Angaben zum Bioverhalten dieser Komplexe, insbesondere Aussagen zur Herzaffinität fehlen.
Zur Beurteilung der Durchblutung des Herzmuskels sind bisher Tc- Isonitrilverbindungen und Tc-DMPE-Verbindungen (DMPE = Bis(dimethylphosphino)ethan) in der klinischen Anwendung.
Das ^{99m}Tc-hexa-MIBI als favorisierter Vertreter der Isonitrilkomplexe [K.McKusick, B.L.Holman, A.G.Jones et al., J.Nucl.Med. 27 (1986) 878] zeichnet sich durch eine hohe und konstante Herzaufnahrne aus, nachteilig ist die anfängliche Speicherung in der Lunge.
^{99m}Tc-DPO als Klinisch angewandte DMPE-Verbindung [W.Mohnike, J.Schmidt, F.Uhlich, S.Seifert, R.Syhre, R.Münze, NucCompact 18 (1987) 184] wird schnell aus dem Blut extrahiert, ungünstig wirkt sich jedoch seine hohe konstante Leberaufnahme aus. Dies trifft auch auf kationische Technetiumkomplexe zu, die sich von ethersubstituierten Diphosphinen und Diarsin ableiten [EP 0 337 654 und EP 0 311 352].
^{99m}Tc-BATO-Komplexe [A.D.Nunn, E.N.Treher, T.Feld, J.Nucl.Med. 27 (1986) 893] und verschiedene ^{99m}Tc-Arene [D.w.Wester, D.L.Nosco, J.R.Coveney, R.T.Dean J.Nucl.Med. 27 (1986) 894] befinden sich in der klinischen Testung. Sie konnten sich für eine routinemäßige Anwendung bisher nicht durchsetzen.

Bei der Konzipierung neuer Komplexverbindungen des Technetiums ist von Bedeutung, daß Technetium als Übergangsmetall eine hohe Affinität zu weichen Donoratomen hat. So sind vorzugsweise Komplexbildner mit Schwefel- und Phosphoratomen zur Bildung von Komplexverbindungen mit Technetium geeignet.
Bei schwefelhaltigen Komplexbildnern dominieren solche mit Thiolgruppen. Umsetzungen von Technetium mit Komplexbildnern, die Thioethergruppen enthalten, sind in [G.F.E.Morgan, J.Pope, J.R.Thornback, A.E.Theobald, 2. Intern.Symp. on Technetium, Padua 1986, 65 ; S.Truffer, E.Ianoz, P.Lerch, M.Kosinski, Inorg.Chim. Acta 149 (1988) 217 ; N.Bryson, J.C.Dewan, J.Lister-Lames, A.G.Jones, A.Davison, Inorg.Chem. 27 (1988) 2154] beschrieben. Von den Tc-Komplexen dieser Komplexbildner, die außer einer oder mehrereren Thioethergruppen Aminogruppen als weitere komplexbildende Einheiten enthalten, ist jedoch keine Myokardaffinität bekannt.
Tridentate Dithiole mit einer Ether- oder Thioethergruppe ergeben in Gegenwart eines zweiten, monodentaten Thiols nur neutrale Komplexe des Technetium(V) [H.-J.Pietzsch, H.Spies, S.Hoffmann, Inorg.Chim.Acta 165 (1989) 163] und sind somit nicht myokardaffin.

Der Erfindung liegt die Aufgabe zugrunde, einfach positiv geladene lipophile Technetiumkomplexe zu finden, die bevorzugt im Herzmuskel angereichert werden und eine niedrige Akkumulationsrate in benachbarten Organen wie z.B der Leber aufweisen, und damit zur Diagnostik von Myokardschäden dienen können. Die Aufgabe schließt ein Verfahren zur Herstellung der gefundenen Komplexe mit ein.

Die Aufgabe wird erfindungsgemäß mit einfach positiv geladenen myokardgängigen Technetiumkomplexverbindungen der allgemeinen Formel [TcL¹ₐL²_{b}]⁺, mit a = 1,2,3,4 und b = 2,3 , gelöst, in der der Ligand L¹ ein mehrzähniges Sulfid oder ein mehrzähniger Ether der allgemeinen Formel

R¹-X-[(CR²R³)m-Y]ₙ-(CR²R³)ₘ-Z-R⁴

mit X, Y, Z = O, S, oder NR⁵,
wobei mindestens eine Thioether- oder Ethergruppierung im Molekül vorhanden sein muß,
und R¹... R⁴ = H, Alkyl, Cycloalkyl, Aralkyl, Aryl, Heteroaryl (für R¹R⁴ = H ist n>2)
oder CH₂COOR⁵, CH₂CN, CH₂OR⁵ mit R⁵ = H oder Alkyl.
wobei R¹R⁴ bzw. (CR²R³)m auch ein Alkylen-. Cycloalkylen oder Arylenrest sein kann
und m = 2,3, bzw. n = O,1,2,3,4
ist, und der Ligand L² ein organischer, monodentater, neutraler oder basischer Komplexbildner, der allgemeinen Formel R⁶-A mit A = OH, SH, NH₂, NR⁷₂, SR⁷, PR⁷₂, -NC
und R⁶, R⁷ = H, Alkyl, Aryl, Cycloalkyl, Heteroaryl ist.

Die Kombination des mehrzähnigen und des monodentaten Komplexbildners wird so gewählt, daß die von der Summe der Donoratome auf das Metall übertragene Ladung eine Einheit niedriger ist als die Ladungszahl des Technetiums (beispielsweise 2 für Tc(III) oder 0 für (Tc(I)). Dabei können sowohl der Thioether als auch der einzähnige Komplexbildner Träger der negativen Ladung sein.
Vorteilhafterweise wird als Komplexbildner ein Gemisch, bestehend aus einem mehrzähnigen Thioether und einem monodentaten Thiol eingesetzt.
Bezüglich des Verfahrens wird die Aufgabe dadurch gelöst, daß der Komplexbildner L¹ mit dem zweiten Komplexbildner L² entweder gleichzeitig im Gemisch oder in einer nachfolgenden Reaktion in saurer, wäßriger oder wäßrig-alkoholischer Lösung bei Normaltemperatur mit einer Technetiumverbindung und einem Reduktionsmittel umgesetzt wird.
Das Verfahren wird vorzugsweise so durchgeführt, daß Technetat(VII) in organisch/wäßriger Lösung in Gegenwart eines Gemisches beider Komplexbildner, gelöst in einem mit Wasser mischbaren organischen Solvens, mit überschüssigem Zinn(II)-chlorid versetzt wird.
Die Reduktion kann auch durch den Komplexbildner selbst erfolgen, sofern er über reduzierende Gruppen verfügt.
Die resultierenden Verbindungen können mit einem organischen Lösungsmittel aus der wäßrigen Phase extrahiert werden.
Die mehrzähnigen Komplexbildner L¹ sind beispielweise offenkettige, neutrale tetradentate, oder neutrale bidentate, oder zweibasische tetradentate oder cyclische neutrale tetradentate Thioether bzw.
deren Sauerstoffanaloge. Der Schwefel oder Sauerstoff kann teilweise durch Stickstoff im Molekül ersetzt werden, der Komplexbildner muß jedoch mindestens eine Thioether- oder Ethergruppierung enthalten.
Der zusätzliche monodentate Komplexbildner L² ist ein organischer monodentater neutraler oder basischer Komplexbildner, z.B ein Thiol, ein substituiertes Phosphin , ein Thioether oder ein substituiertes Isonitril.
Alle o.g. Restgruppen sind sowohl im mehrzähnigen als auch im einzähnigen Komplexbildner weitgehend variabel und umfaßen auch Reste mit funktionellen Gruppen, wie beispielsweise Ester-, Ether- oder Aminogruppen.
Die Erfindung ist insofern vorteilhaft, daß durch die Kombination zweier unterschiedlicher Komplexbildner eine große Anzahl unterschiedlicher Verbindungen darstellbar ist, die aufgrund ihrer speziellen chemischen Eigenschaften potentielle Radiopharmaka zur Untersuchung des Herzmuskels darstellen. Die Variierbarkeit wird dadurch weiter erhöht, daß jeder Komplexbildner durch Substitution an beliebigen Restgruppen veränderlich ist. Es können auch reaktive Gruppen, wie beispielsweise hydrolysierbare Estergruppen eingeführt werden, was bzgl. des biologischen Verhaltens der Verbindungen von Vorteil sein kann.

Nachfolgend soll die Erfindung anhand einiger konkreter Ausführungsformen beispielhaft näher erläutert werden.
Entsprechend der allgemeinen Formeln für die Liganden L¹ und L² sind folgende Varianten als Beispiele zu nennen:
- für den Liganden L¹

   1a: Et-S-CH₂CH₂-O-CH₂CH₂-O-CH₂CH₂-S-Et

   1b: Bu-S-CH₂CH₂-O-CH₂CH₂-O-CH₂CH₂-S-Bu

   1c: Et-O-CH₂CH₂-S-CH₂CH₂-S-CH₂CH₂-O-Et

   1d: Et-S-CH₂CH₂-S-CH₂CH₂-S-CH₂CH₂-S-Et

   1e: Bu-S-CH₂CH₂-S-CH₂CH₂-S-CH₂CH₂-S-Bu
- für den Liganden L²

   2a: EtSH

   2b: iso-Prop-SH

   2c: C₆H₅-SH

   2d: p-CHO₃-C₆H₄-SH

Unter Berücksichtigung der Forderung, daß die von der Summe der Donoratome auf das Metall übertragene Ladung eine Einheit niedriger sein muß als die Ladungszahl des Technetiums, wobei sowohl der Thioether als auch der einzähnige Komplexbildner Träger der negativen Ladung sein kann, ergeben sich unterschiedliche Kombinationsmöglichkeiten der Liganden L¹ und L², von denen folgende explicit ausgeführt werden sollen:

A 1d+ 2b = Bis(isopropylthiolato)(3,6,9,12 - tetrathiatetradecan)technetium(III)

B 1d + 2c = Bis(thiophenolato)(3,6,9,12-tetrathiatetradecan)technetium(III)

C 1d + 2c = Bis(para-methoxythiophenolato) (3,6,9,12 - tetrathiatetradecan)technetium(III)

D 1e + 2c = Bis(thiophenolato)-5,8,11,14-tetrathiaoctadecan)technetium(III)

Um diese beispielhaft genannten Zielverbindungen unter Einbeziehung des erfindungsgemäßen Verfahrens für Bioverteilungsuntersuchungen herzustellen , wird beispielsweise wie folgt verfahren :
3 mg des Liganden L¹ ( TTTD ) und 5 mg des Liganden L² (Isopropylmercaptan ) werden in 4 ml Aceton gelöst. Dazu gibt man 1.6 »mol ⁹⁹Tc-TcO₄- und 2 ml ^{99m}Tc-TcO₄-Generatoreluat. Nach Zugabe von 2 mg SnCl₂*2H₂O in 100 »l 0.1 N HCl wird die Lösung mit 3 ml Chloroform extrahiert.

Das Lösungsmittel wird im Stickstoffstrom abgezogen und der Rückstand in 0.5 ml Ethanol aufgenommen. Anschließend wird mit 4 ml Propylenglykol aufgefüllt, filtriert und mit 1.5 ml Wasser versetzt. Die Lösung ist injektionsfertig .
Nach dieser Verfahrensweise werden bei Verwendung der o.g. Ligandenkombinationen die injektionsfertigen Präparate A bis D hergestellt.
Diese Präparate zeigen in Tierverteilungsstudien an Ratten die in der Tabelle dargestellte deutliche Aufnahme in den Herzmuskel.

| Tab.: Organverteilungswerte nach i.v.-Injektion an Wistar-Ratten | | | | | |
|---|---|---|---|---|---|
| Organ | Zeit p.i. [min.] | Testpräparat [% Dosis/g] | | | |
| | | A | B | C | D |
| Herz | 5 | 2.5 | 1.8 | 2.3 | 1.2 |
| | 30 | 2.2 | 1.6 | 2.0 | 0.6 |
| | 60 | 2.0 | 1.5 | 1.9 | 0.6 |
| Blut | 5 | 1.6 | 1.2 | 3.4 | 0.7 |
| | 30 | 1.2 | 0.7 | 2.2 | 0.6 |
| | 60 | 0.6 | 0.5 | 1.7 | 0.6 |
| Leber | 5 | 2.5 | 3.9 | 4.0 | 5.6 |
| | 30 | 2.0 | 2.2 | 2.5 | 3.5 |
| | 60 | 1.5 | 2.0 | 2.1 | 2.5 |

Autoradiographische Aufnahmen vom Rattenherzen zeigen eine Aktivitätsspeicherung bevorzugt im linksventrikulären Myokard in gutem Kontrast zu den Herzinnenräumen.
Um eine eindeutige chemisch-physikalische Charakterisierung der erfindungsgemäßen Verbindungen vornehmen zu können, ist deren Darstellung in kristalliner Form erforderlich. Eine solche Präparation kann z.B. folgendermaßen erfolgen:
30 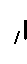 mg (148 »mol) KTcO₄, gelöst in 5 ml Wasser, werden mit 60 mg (222 umol) des Liganden L¹ [3,6,9,12-Tetrathiatetradecan (TTTD)] und 34 mg (444 »mol) des Liganden L² [Isopropylmercaptan], gelöst in 8 ml Aceton, versetzt.

Nach Zugabe einer Lösung von 170 mg SnCl₂*2H₂O in 0.5 ml 0.1 N HCl färbt sich die Reaktionsmischung tiefrot. Die Lösung wird 20 Minuten bei Raumtemperatur gerührt und anschliessend zur Trockne gebracht. Der Rückstand wird zur Entfernung des überschüssigen Komplexbildners mit 3x5 ml Ether extrahiert, dann in 4 ml Aceton aufgenommen, ungelöstes Produkt abfiltriert und die Lösung mit 6 ml Wasser versetzt.
Zu dieser Lösung werden im Verlauf von 10 Minuten unter Rühren insgesamt 50 mg KPF₆ gegeben.
Das ausgefallene tiefrote Produkt wird abgetrennt, zunächst mit 3x1 ml Wasser, dann mit 5x1 ml Ether gewaschen, in 3 ml Chloroform aufgenommen und mit 3 ml Ether versetzt. Der Komplex kristallisiert in Form schwarzer kompakter Kristalle.
Ausbeute: 41 mg (42 %)
Die Elementaranalyse (C,H,S,Tc) entspricht der Zusammensetzung [Tc(TTTD)(SR)₂]PF₆ [Bis(isopropylthiolato)(3,6,9,12-tetrathiatetradecan)technetium(III)hexafluorophosphat]. Die Gegenwart sowohl des mehrzähnigen Komplexbildners als auch des Monothiols im Komplex werden durch IR- und ¹H-NMR-Daten bestätigt.

Unter Verwendung der o.g. weiteren Möglichkeiten der Kombination der Liganden L¹ und L² werden analog Bis(thiophenolato)(3,6,9,12-tetrathiatetradecan)-technetium(III)hexafluorophosphat und Bis(para-methoxythiophenolato)(3,6,9,12-tetrathiatetradecan)technetium(III)hexafluorophosphat sowie Bis(thiophenolato)5,8,11,14-tetrathiaoctadekan)technetium(III)hexafluorophosphat hergestellt und spektroskopisch charakterisiert.

## Patentansprüche

1. Einfach positiv geladene myokardgängige Technetiumkomplexverbindungen der allgemeinen Formel [TcL¹ₐL²_{b}]⁺, mit a = 1,2,3,4 und b = 2,3, gekennzeichnet dadurch, daß L¹ ein mehrzähniges Sulfid oder ein mehrzähniger Ether der allgemeinen Formel
R¹-X-[(CR²R³)m-Y]ₙ-(CR²R³)ₘ-Z-R⁴
mit X, Y, Z = O, S, oder NR⁵,
wobei mindestens eine Thioether oder Ethergruppierung im Molekül vorhanden sein muß,
und R¹... R⁴ = H, Alkyl, Cycloalkyl, Aralkyl, Aryl, Heteroaryl (für R¹R⁴ = H ist n>2)
oder CH₂COOR⁵, CH₂CN, CH₂OR⁵, mit R⁵ = H oder Alkyl,
wobei R¹R⁴ bzw. (CR²R³)m auch ein Alkylen-, Cycloalkylen oder Arylenrest sein kann
und m = 2,3, bzw. n = O,1,2,3,4
ist,und L² ein organischer, monodentater, neutraler oder basischer Komplexbildner, der allgemeinen Formel R⁶-A
mit A = OH, SH, NH₂, NR⁷₂, SR⁷, PR⁷₂, -NC
und R⁶, R⁷ = H, Alkyl, Aryl, Cycloalkyl, Heteroaryl
ist.

2. Myokardgängige Komplexverbindung nach Anspruch 1, dadurch gekennzeichnet, daß die Komplexbildner L¹ bzw. L² ein mehrzähniger Thioether und ein monodentates Thiol sind.

3. Verfahren zur Herstellung von Komplexverbindungen nach Anspruch 1, dadurch gekennzeichnet. daß der Komplexbildner L¹ mit dem zweiten Komplexbildner L² entweder gleichzeitig im Gemisch oder in einer nachfolgenden Reaktion in saurer, wäßriger oder wäßrig-alkoholischer Lösung bei Normaltemperatur mit einer Technetiumverbindung und einem Reduktionsmittel umgesetzt wird.

## Claims

1. Positively charged myocardial-path technetium complex compounds of the general formula [TcL¹ₐL²_{b}]⁺ with a = 1,2,3,4 and b = 2,3, wherein L¹ is a multidentate sulfid or a multidentate ether of the general formula
R¹-X-[(CR²R³)ₘ-Y]ₙ-(CR²R³)ₘ-Z-R⁴
wherein X,Y,Z = O,S or NR⁵ with at least one thioether or ether group in the molecule,
and R¹.......R⁴ = H, alkyl, cycloalkyl, aralkyl, aryl, heteroaryl (for R¹R⁴ = H is n>2) or CH₂COOR⁵, CH₂CN, CH₂OR⁵, with R⁵ = H or alkyl,
wherein R¹R⁴ and (CR²R³) resp. may be also an alkylene-, cycloalkylene or arylen moiety and m = 2,3 and n = 0,1,2,3,4
and L² is an organic, monodentate, neutral or basic ligand of the general formula
R⁶-A
with A = OH, SH, NH₂, NR⁷₂, SR⁷, PR⁷₂, -NC
and R⁶, R⁷ = H, alkyl, aryl, cycloalkyl, heteroalkyl

2. Myocardial-path complex compounds as claimed in claim 1, wherein the ligands L¹ and L² are a multidentate thioether and a monodentate thiol.

3. Process for the production of complex compounds as claimed in claim 1, characterized by the fact, that the ligand L¹ and the second ligand L² react with a technetium compound and a reduction agent, either simultaneously in the mixture or in a subsequent reaction in acidic, aqueous or aqueous-alcoholic solution at ambient temperature.

## Revendications

1. Composés complexes de technetium du trajet myocardique chargés simplement positivement de la formule générale [TcL¹ₐL²_{b}]⁺, avec a = 1,2,3,4 et b = 2,3 caractérisés en ce que L¹ est un sulfure à plusieurs dents ou une éther à plusieurs dents de la formule générale
R¹-X-[(CR²R³)m-Y]ₙ-(CR²R³)ₘ-Z-R⁴
avec X, Y, Z = O, S, ou NR⁵,
un thioéther ou groupe d'éther au moins devant exister dans la molécule,
et R¹....R⁴ = H, alcoyle, cycloalcoyle, aralcoyle, aryle, hétéroaryle
(pour R¹R⁴ = H est n>2)
ou CH₂COOR⁵, CH₂CN, CH₂OR⁵, avec R⁵ = H ou alcoyle,
R¹R⁴ voire (CR²R³)m pouvant également être un alcoylène, un
cycloalcoylène ou reste d'arylène et m = 2,3 voire n = 0,1,2,3,4
et que L² est un formateur complexe organique, monodental, neutre ou basique de la formule générale R⁶-A
avec A = OH,SH,NH₂,NR⁷₂,SR⁷,PR⁷₂,-NC
et R⁶, R⁷ = H, alcoyle, aryle, cycloalcoyle, hétéroaryle.

2. Composés complexes de technetium du trajet myocardique selon la revendication 1 caractérisés en ce que les formateurs complexes L¹ voire L² sont un thioéther à plusieurs dents et un thiol monodental.

3. Procédé de fabrication de composés complexes de technetium du trajet myocardique selon la revendication 1 caractérisé en ce que le formateur complexe L¹ est appliqué avec le deuxième formateur complexe L² soit en même temps dans le mélange soit dans une réaction consécutive dans une solution acide, aqueuse ou aqueuse-alcoolisée pour une température normale avec un composé de technetium et un agent de réduction.
